# EUROPÄISCHE PATENTSCHRIFT

(11) **EP 1 002 032 B1**
(45) Veröffentlichungstag und Bekanntmachung des Hinweises auf die Patenterteilung: **17.09.2003**
(21) Anmeldenummer: 98940182.3
(22) Anmeldetag: 08.07.1998
(51) Int. Cl.: C11D 1/62, A61K 7/50, C11D 1/94, C11D 1/835, C11D 3/22, C11D 3/382

(54) **DETERGENSGEMISCHE ENTHALTEND ESTERQUATS, CHITOSAN UND/ODER CHITOSANDERIVATE UND PROTEINHYDROLYSATE**
DETERGENT MIXTURES CONTAINING ESTER QUATS, CHITOSANE AND/OR CHITOSANE DERIVATIVES AND PROTEIN HYDROLYZATES
MELANGES DETERGENTS CONTENANT DES ESTERS QUATERNAIRES, DE LA CHITOSANE ET/OU DES DERIVES DE CHITOSANE ET DES HYDROLYSATS DE PROTEINE

(30) Priorität: 17.07.1997 DE 19730649
(43) Veröffentlichungstag der Anmeldung: 24.05.2000
(73) Patentinhaber: Cognis Deutschland GmbH & Co. KG, 40589 Düsseldorf (DE)
(72) Erfinder: WEBER, Peter, D-40789 Monheim (DE); FABRY, Bernd, D-41352 Korschenbroich (DE)
(86) Internationale Anmeldenummer: EP9804244
(87) Internationale Veröffentlichungsnummer: WO99003959

(56) Entgegenhaltungen:
- WO-A-94/16677
- WO-A-95/05802
- WO-A-97/06780
- WO-A-97/18033
- DE-A- 4 442 987
- DE-A- 19 604 180
- ULLMANN'S ENCYCLOPEDIA, Band 6, 1986, Seiten 231-232

## Beschreibung

### Gebiet der Erfindung

Die Erfindung betrifft Detergensgemische mit einem Gehalt an Esterquats, Chitosanen und Proteinhydrolysaten sowie gegebenenfalls Alkylglykosiden und/oder Betainen sowie die Verwendung der Gemische zur Herstellung von oberflächenaktiven Mitteln.

### Stand der Technik

Aus dem Stand der Technik sind eine Vielzahl von Tensidmischungen bekannt, die Einsatz in den unterschiedlichsten Gebieten finden. Im Bereich der Waschmittelrohstoffe und Kosmetika gibt es jedoch ein gleichartiges Bedürfnis nach möglichst konzentrierten Tensidvorgemischen, die sich durch gute Reinigungs- und Avivageeigenschaften auszeichnen, wobei dies einmal synthetische Fasern, also Textilien und deren Vorprodukte, und zum anderen natürliche (Keratin-)Fasem, also menschliches Haar betrifft. Eine weitere Forderung besteht darin, daß die Produkte über eine optimale Hautverträglichkeit verfügen, so daß die Gefahr, daß selbst besonders sensibilisierte Verbraucher entweder im direkten Umgang oder indirekt über den Kontakt mit der behandelten Faser Hautirritationen erleiden, praktisch ausgeschlossen ist.

WO-A-9 505 802 beschreibt Tensidgemische enthaltend Proteinhydrolysate und kationische monomere Tenside wie beispielsweise Esterquats.

Die komplexe Aufgabe der Erfindung hat demnach darin bestanden, neue Detergensgemische sowohl für die Waschmittel- als auch für die Kosmetikindustrie zur Verfügung zu stellen, die sich gleichzeitig durch eine besonders hohe Hautverträglichkeit, ein gutes Haut- und Textilreinigungs- und Wiederbenetzungsvermögen sowie ausgezeichnete Avivageeigenschaften für synthetische und natürliche Fasern auszeichnen sollten.

### Beschreibung der Erfindung

Gegenstand der Erfindung sind Detergensgemische, enthaltend
(a) Esterquats,
(b) Chitosan und/oder Chitosanderivate und
(c) Proteinhydrolysate.

Überraschenderweise wurde gefunden, daß die erfindungsgemäßen Detergensgemische nicht nur besonders gut hautverträglich sind, sondern zudem über ein besonders hohes Reinigungsvermögen sowohl für Textilien als auch für Haut und Haare verfügen. Textilien wie Haaren verleihen sie ferner nicht nur einen angenehmen Weichgriff, sie erniedrigen auch die statische Aufladung zwischen den Fasern.

### Esterquats

Unter der Bezeichnung "Esterquats" (Komponente a) werden im allgemeinen quatemierte Fettsäuretriethanolaminester-salze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Übersichten zu diesem Thema sind beispielsweise von R.Puchta et al. in **Tens. Surf. Det., 30, 186 (1993),** M.Brock in **Tens. Surf. Det. 30, 394 (1993),** R.Lagerman et al. in J. **Am. Oil. Chem. Soc., 71, 97 (1994)** sowie l.Shapiro in **Cosm.Toil. 109, 77 (1994)** erschienen.

Die quaternierten Fettsäuretriethanolaminestersalze folgen der Formel (I), 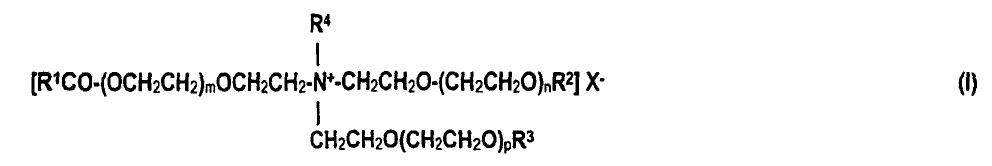 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäure-reiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 :1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab. Aus anwendungstechnischer Sicht haben sich quaternierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quatemierten Fettsäuretriethanolaminestersalzen kommen als Esterquats ferner auch quatemierte Estersalze von Fettsäuren mit Diethanolalkylaminen der Formel **(II)** in Betracht, 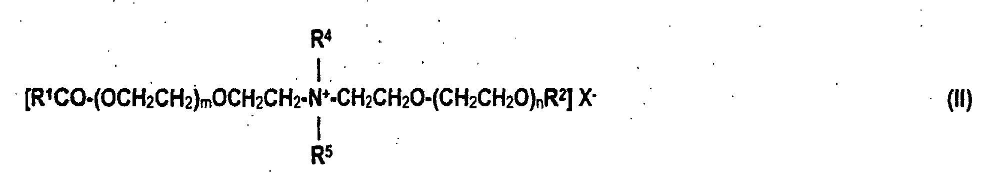 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quatemierten Estersalze von Fettsäuren mit 1,2-Dihydroxypropyldialkylaminen der Formel **(III)** zu nennen, 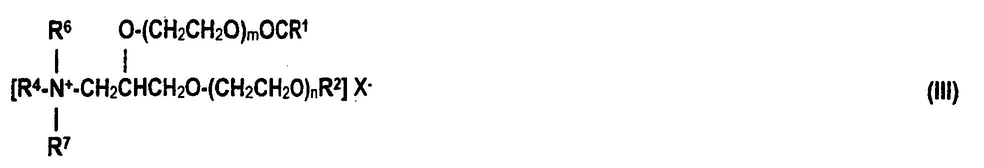 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III).** Üblicherweise gelangen die Esterquats in Form 50 bis 90 Gew.-%iger alkoholischer Lösungen in den Handel, die bei Bedarf problemlos mit Wasser verdünnt werden können. Es ist femer ebenfalls möglich, die Esterquats zusammen mit Fettalkoholen in Form von Schuppen einzusetzen, wie dies beispielsweise in der Deutschen Patentschrift **DE-C1 4308794** (Henkel) beschrieben wird.

### Chitosane und Chitosanderivate

Chitosane (Komponente b) stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

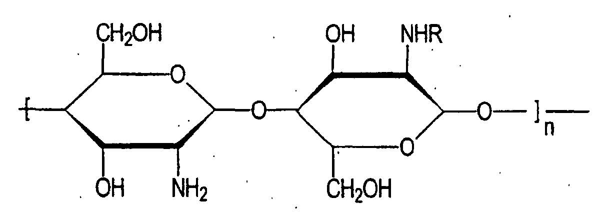

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim,** **Verlag Chemie, 1986, S. 231-332).** Übersichten zu diesem Thema sind auch beispielsweise von B.Gesslein et al. in **HAPPI 27, 57 (1990),** O.Skaugrud in **Drug Cosm.lnd. 148, 24 (1991)** und E.Onsoyen et al. in **Seifen-Öle-Fette-Wachse 117, 633 (1991)** erschienen. Zur Herstellung der Chitösane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR-A 2701266** bekannt. Vorzugsweise werden solche Typen eingesetzt, wie sie in den deutschen Patentanmeldungen **DE-A1 4442987** und **DE-A1 19537001** (Henkel) offenbart werden, und die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch anionisch bzw. nichtionisch derivatisierte Chitosane, wie z.B. Carboxylierungs-, Succinylierungs- oder Alkoxylierungsprodukte in Frage, wie sie beispielsweise in der deutschen Patentschrift **DE-C2 3713099** (L'Oréal) sowie der deutschen Patentanmeldung **DE-A1 19604180** (Henkel) beschrieben werden.

### Proteinhydrolysate

Proteinhydrolysate (Komponente c) stellen Abbauprodukte von tierischen oder pflanzlichen Proteinen, beispielsweise Collagen, Elastin oder Keratin und vorzugsweise Mandel- und Kartoffelprotein sowie insbesondere Weizen-, Reis- und Sojaprotein dar, die durch saure, alkalische und/oder enzymatische Hydrolyse gespalten werden und danach ein durchschnittliches Molekulargewicht im Bereich von 600 bis 4000, vorzugsweise 2000 bis 3500 aufweisen. Obschon Proteinhydrolysate in Ermangelung eines hydrophoben Restes keine Tenside im klassischen Sinne darstellen, finden sie wegen ihrer dispergierenden Eigenschaften vielfach Verwendung zur Formulierung oberflächenaktiver Mittel. Übersichten zu Herstellung und Verwendung von Proteinhydrolysaten sind beispielsweise von G.Schuster und A.Domsch in **Seifen Öle Fette Wachse, 108, 177 (1982)** bzw. **Cosm.Toil. 99, 63 (1984),** von H.W.Steisslinger in **Parf.Kosm. 72, 556 (1991)** und F.Aurich et al. in **Tens.Surf.Det. 29, 389 (1992)** erschienen. Vorzugsweise werden pflanzliche Proteinhydrolysate auf Basis von Weizengluten oder Reisprotein eingesetzt, deren Herstellung in den beiden Deutschen Patentschriften **DE-C1 19502167** und **DE-C1 19502168** (Henkel) beschrieben wird.

### Alkyl- und/oder Alkenyloligoglykoside

In einer bevorzugten Ausführungsform enthalten die erfindungsgemäßen Detergensgemische weiterhin als Komponente (d) Alkyl- und Alkenyloligoglykoside, die der Formel **(IV)** folgen,

**R**^{**8**}**O-[G]**_{**p**} **(IV)**

in der R⁸ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0301298** und **WO 90/03977** verwiesen. Die Alkylund/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside.** Die Indexzahl p in der allgemeinen Formel **(IV)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁸ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁸ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palm-oleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Betaine

In einer weiteren bevorzugten Ausführungsform können die erfindungsgemäßen Zubereitungen weiterhin als Komponente (e) Tenside vom Betaintyp enthalten. Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Femer ist auch die Anlagerung von ungesättigten Carbonsäuren wie beispielsweise Acrylsäure möglich. Zur Nomenklatur und insbesondere zur Unterscheidung zwischen Betainen und "echten" Amphotensiden sei auf den Beitrag von U.Ploog in **Seifen-Öle-Fette-Wachse, 198, 373 (1982)** verwiesen. Weitere Übersichten zu diesem Thema finden sich beispielsweise von A.O'Lennick et al. in **HAPPI, Nov. 70 (1986),** S.Holzman et al. in **Tens.Surf.Det. 23, 309 (1986),** R.Bibo et al. in **Soap Cosm.Chem.Spec. Apr. 46 (1990)** und P.Ellis et al. in **Euro Cosm. 1, 14 (1994).** Beispiele für geeignete Betaine stellen die Carboxyalkylierungsprodukte von sekundären und insbesondere tertiären Aminen dar, die der Formel **(V)** folgen, 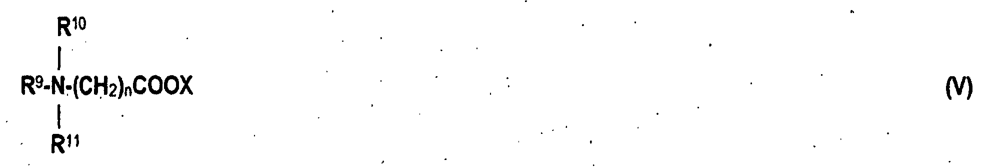 in der R⁹ für Alkyl- und/oder Alkenylreste mit 6 bis 22 Kohlenstoffatomen, R¹⁰ für Wasserstoff oder Alkylreste mit 1 bis 4 Kohlenstoffatomen, R¹¹ für Alkylreste mit 1 bis 4 Kohlenstoffatomen, n für Zahlen von 1 bis 6 und X für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von **Amidoaminen** in Betracht, die der Formel **(VI)** folgen, 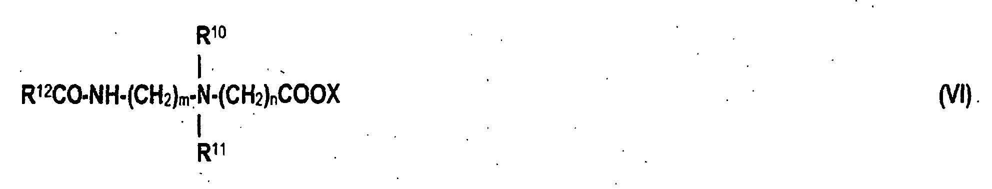 in der R¹²CO für einen aliphatischen Acylrest mit 6 bis 22 Kohlenstoffatomen und 0 oder 1 bis 3 Doppelbindungen, m für Zahlen von 1 bis 3 steht und R¹⁰, R¹¹, n und X die oben angegebenen Bedeutungen haben. Typische Beispiele sind Umsetzungsprodukte von Fettsäuren mit 6 bis 22 Kohlenstoffatomen, namentlich Capronsäure, Capryisäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethyl-aminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

### Detergensgemische

In einer bevorzugten Ausführungsform der Erfindung werden die Detergensgemische in Form von wäßrigen Zubereitungen mit einem Feststoffgehalt im Bereich von 15 bis 70, vorzugsweise 25 bis 50 und insbesondere 35 bis 45 Gew.-% eingesetzt. Bezogen auf den Feststoffgehalt können die Gemische die Komponenten (a) bis (e) in den folgenden Mengen enthalten:

| | |
|---|---|
| (a) | 10 bis 60, vorzugsweise 20 bis 40 Gew.-% Esterquats, |
| (b) | 1 bis 10, vorzugsweise 2 bis 5 Gew.-% Chitosan und/oder Chitosanderivate, |
| (c) | 10 bis 30, vorzugsweise 5 bis 20 Gew.-% Proteinhydrolysate, |
| (d) | 0 bis 25, vorzugsweise 5 bis 20 Gew.-% Alkyl- und/oder Alkenyloligoglykoside und |
| (e) | 0 bis 25, vorzugsweise 5 bis 20 Gew.-% Betaine, |

mit der Maßgabe, daß sich die Gewichtsangaben jeweils zu 100 Gew.-% ergänzen.

### Gewerbliche Anwendbarkeit

Die erfindungsgemäßen Detergensgemische verfügen über ein ausgezeichnetes Reinigungsvermögen und verleihen synthetischen wie natürlichen Fasern einen angenehmen Weichgriff; zudem erniedrigen sie die elektrostatische Aufladung zwischen den Fasern und verbessern deren Wieder-benetzbarkeit. Ein weiterer Gegenstand der Erfindung betrifft daher die Verwendung der Gemische zur Herstellung von oberflächenaktiven Zubereitungen, wie beispielsweise Wasch-, Spül-, Reinigungs- und Avivagemitteln sowie kosmetischen Zubereitungen wie insbesondere Mitteln zur Haar- und Körperpflege.

### Wasch-, Spül- und Reinigungsmittel

Typische Beispiele für Wasch-, Spül- und Reinigungsmittel, die die erfindungsgemäßen Detergensgemische enthalten können, sind flüssige bis pastöse Textilweichspülmittel, Handgeschirrspülmittel, maschinelle Geschirrspülmittel, Klarspülmittel sowie Universal-, Haushalts- und Sanitärreiniger, pulverförmige bzw. granulierte Universalwaschmittel sowie insbesondere Avivagemittel. Die Zubereitungen können weitere typische Inhaltsstoffe wie beispielsweise Waschmitteltenside, Builder, Enzyme, Enzymstabilisatoren, Bleichmittel, optische Aufheller, Verdickungsmittel, Soil repellants, Schauminhibitoren, Lösungsvermittler, anorganische Salze sowie Duft-und Farbstoffe aufweisen.

Typische **Waschmitteltenside** sind Alkylbenzolsulfonate, Alkylsulfate, Sulfofettsäuren, Sulfofettsäuremethylester und dergleichen. Geeignete **Builder** sind Zeolithe, Schichtsilicate, Phosphate sowie Ethylendiamintetraessigsäure, Nitrilotriessigsäure, Citronensäure sowie anorganische Phosphonsäuren.

Unter den als Peroxy-**Bleichmittel** dienenden Verbindungen haben das Natriumperborat-Tetrahydrat und das Natriumperborat-Monohydrat eine besondere Bedeutung. Weitere Bleichmittel sind beispielsweise Peroxycarbonat, Citratperhydrate sowie H₂O₂-liefernde persaure Salze der Persäuren wie Perbenzoate, Peroxyphthalate oder Diperoxydodecandisäure. Sie werden üblicherweise in Mengen von 8 bis 25 Gew.-% eingesetzt. Bevorzugt ist der Einsatz von Natriumperborat-Monohydrat in Mengen von 10 bis 20 Gew.-% und insbesondere von 10 bis 15 Gew.-%. Durch seine Fähigkeit, unter Ausbildung des Tetrahydrats freies Wasser binden zu können , trägt es zur Erhöhung der Stabilität des Mittels bei.

Als **Verdickungsmittel** können beispielsweise gehärtetes Rizinusöl, Salze von langkettigen Fettsäuren, die vorzugsweise in Mengen von 0 bis 5 Gew.-% und insbesondere in Mengen von 0,5 bis 2 Gew.-%, beispielsweise Natrium-, Kalium-, Aluminium-, Magnesium- und Titanstearate oder die Natrium- und/oder Kaliumsalze der Behensäure, sowie weitere polymere Verbindungen eingesetzt werden. Zu den letzten gehören bevorzugt Polyvinylpyrrolidon, Urethane und die Salze polymerer Polycarboxylate, beispielsweise homopolymerer oder copolymerer Polyacrylate, Polymethacrylate und insbesondere Copolymere der Acrylsäure mit Maleinsäure, vorzugsweise solche aus 50 bis 10% Maleinsäure. Die relative Molekülmasse der Homopolymeren liegt im allgemeinen zwischen 1000 und 100000, die der Copolymeren zwischen 2000 und 200000, vorzugsweise zwischen 50000 bis 120000, bezogen auf die freie Säure. Insbesondere sind auch wasserlösliche Polyacrylate geeignet, die beispielsweise mit etwa 1% eines Polyallylethers der Sucrose quervernetzt sind und die eine relative Molekülmasse oberhalb 1000000 besitzen Beispiele hierfür sind unter dem Namen Carbopol® 940 und 941 erhältliche Polymere. Die quervernetzten Polyacrylate werden vorzugsweise in Mengen nicht über 1 Gew.-% besonders bevorzugt in Mengen von 0,2 bis 0,7 Gew.-% eingesetzt.

Als **Enzyme** kommen solche aus der Klasse der Proteasen, Lipase, Amylasen, Cellulasen bzw. deren Gemische in Frage. Besonders gut geeignet sind aus Bakterienstämmen oder Pilzen, wie Bacillus subtilis, Bacillus lichenformis und Strptomyces griseus gewonnene enzymatische Wirkstoffe. Vorzugsweise werden Proteasen vom Subtilisin-Typ und insbesondere Proteasen, die aus Bacillus lentes gewonnen werden, eingesetzt. Ihr Anteil kann etwa 0,2 bis 2 Gew.-% betragen. Die Enzyme können an Trägerstoffen adsorbiert oder in Hüllsubstanzen eingebettet sein, um sie gegen vorzeitige Zersetzung zu schützen.

Zusätzlich zu mono- und polyfunktionellen Alkoholen und Phosphonaten können die Mittel weitere **Enzymstabilisatoren** enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2 Gew.-%, bezogen auf das Enzym, stabilisiert sind. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B₄O₇).

Beim Einsatz im maschinellen Waschverfahren kann es von Vorteil sein, den Mitteln übliche **Schauminhibitoren** zuzusetzen. Geeignete Schauminhibitoren enthalten beispielsweise bekannte Organopolysiloxane, Paraffine oder Wachse.

### Kosmetische Zubereitungen

Werden die erfindungsgemäßen Detergensgemische zur Herstellung von kosmetischen Zubereitungen wie beispielsweise Haarshampoos, Haarlotionen, Schaumbäder, Cremes oder Lotionen verwendet, können diese als weitere Hilfs- und Zusatzstoffe milde Tenside, Ölkörper, Emulgatoren, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Fettsäureglucamide, und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Anlagerungsprodukte von 15 bis 60 Mol Ethylenoxid an Ricinusöl und/oder gehärtetes Ricinusöl;
(5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(6) Anlagerungsprodukte von 2 bis 15 Mol Ethylenoxid an Ricinusöl und/oder gehärtetes Ricinusöl;
(7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(8) Trialkylphosphate sowie Mono-, Di- und/oder Tri-PEG-alkylphosphate;
(9) Wollwachsalkohole;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepdxiden mit 12 bis 22 Kohlenstoffatomen mit Fettalkoholen mit 12 bis 22 Kohlenstoffatomen und/oder. Polyolen mit 2 bis 15 Kohlenstoffatomen und 2 bis 10 Hydroxylgruppen; sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quatemierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quatemierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partiatglyceriden in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können femer **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin

Als **Konservierungsmittel** eignen sich beispielsweise Phenoxyethanol, Formaldehydiösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Die Hautverträglichkeit der Detergensgemische wurde entsprechend der OECD-Methode No.404 und der EEC Directive 84/449 EEC, Pt.B.4. bestimmt. Die angegebenen Reizsummenscores wurden aus den nach 24, 48 und 72 h erhaltenen Reizscores gebildet. Dabei wurde der im Vergleichsversuch V1 ermittelte Reizsummenscore zu 100 % gesetzt und die in den übrigen Versuchen erhaltenen Scores zu diesem ins Verhältnis gesetzt.

Zur Bestimmung des **Reinigungsvermögens** wurde verschmutztes Baumwollgewebe (Anschmutzung: Staub/Hautfett) im Launder-o-meter bei 60°C mit 1 g der Zubereitungen und 1 g Zeolith A gewaschen und der Weißgrad (%-Remission) photometrisch gegen Bariumsulfat als Standard bestimmt.

Die Beurteilung des **Weichgriffs** erfolgte durch ein Panel von 6 geschulten Personen, die die gewaschenen Baumwollgewebe auf einer Skala von (1) = sehr weich bis (4) = hart bewerteten.

Die **Hydrophilie,** d.h. Wiederbenetzbarkeit der Gewebe, wurde im bekannten Steighöhentest nach DIN 53924 bestimmt, bei dem man Streifen des Baumwollgewebes von 1 cm Breite in Wasser eintaucht und die Höhe mißt, auf die das Wasser aufgrund der Kapillarkräfte in dem Gewebe innerhalb von 1 min steigt; je größer die Steighöhe um so höher ist auch die Hydrophilie des Gewebes.

Die **Naßkämmbarkeit** wurde an braunem Haar (Alkinco #6634, Strähnenlänge 12 cm, Strähnenmasse 1 g) untersucht. Nach der Nullmessung wurden die Strähnen mit 1000 ml der Formulierungen getränkt. Nach einer Einwirkzeit von 5 min wurden die Strähnen 1 min unter fließendem Wasser (1 l/min 38°C) ausgespült. Die Strähnen wurden erneut vermessen und mit der Nullmessung verglichen. Der Fehler bei den Messungen betrug im Mittel 2 %, die statistische Sicherheit lag bei 99 %.

Die Ergebnisse sind in Tabelle 1 zusammengefaßt. Die Beispiele 1 bis 8 sind erfindungsgemäß, die Beispiele V1 und V2 dienen zum Vergleich.

**Tabelle 1**

| **Zusammensetzung und Performance von Detergensgemischen** | | | | | | | | | | |
|---|---|---|---|---|---|---|---|---|---|---|
| **Zusammensetzung / Performance** | **1** | **2** | **3** | **4** | **5** | **6** | **7** | **8** | **V1** | **V2** |
| Esterquat* | 35 | 35 | 20 | 20 | 20 | 20 | 20 | 20 | - | - |
| Distearyldimethylammonium chloride | - | - | - | - | - | - | - | - | 35 | 20 |
| Chitosan | 1 | - | 1 | 1 | 1 | - | - | - | 1 | 1 |
| Succinyliertes Chitosan | - | 1 | - | - | - | 1 | | 1 1 | - | - |
| Wheat Protein Hydrolysate | 14 | 14 | 9 | 9 | 9 | 9 | 9 | 9 | 14 | 9 |
| Coco Glucosides | - | - | 20 | - | 10 | 20 | - | 10 | - | 20 |
| Cocamidopropyl Betaine | - | - | - | 20 | 10 | - | 20 | 10 | - | - |
| Wasser | ad 100 | | | | | | | | | |
| ***Reizsummenscore [%-rel]*** | 85 | 88 | 76 | 78 | 68 | 77 | 79 | 69 | 100 | 90 |
| ***Weißgrad [%-Rem]*** | 59,7 | 60,1 | 75,8 | 76,5 | 78,2 | 74,4 | 75,0 | 73,9 | 52,5 | 66,8 |
| ***Weichgriff*** | 1,5 | 1,5 | 1,0 | 1,0 | 1,0 | 1,0 | 1,0 | 1,0 | 2,5 | 2,5 |
| ***Hydrophilie [mm]*** | 11 | 12 | 11 | 13 | 12 | 11 | 11 | 11 | 8 | 9 |
| ***Naßkämmbarkeit [mV]*** | 44,3 | 45,7 | 50,1 | 51,0 | 49,8 | 49,7 | 49,5 | 50,0 | 36,1 | 37,0 |

| | | | | | | | | | | |
|---|---|---|---|---|---|---|---|---|---|---|
| *) Methylquaternierter Dipalmfettsäuretriethanolaminester, Methylsulfat-Salz | | | | | | | | | | |

## Patentansprüche

1. Detergensgemische, enthaltend
(a) Esterquats,
(b) Chitosan und/oder Chitosanderivate und
(c) Proteinhydrolysate.

2. Detergensgemische nach Anspruch 1, **dadurch gekennzeichnet, daß** sie Esterquats der Formel
**(I)** enthalten, 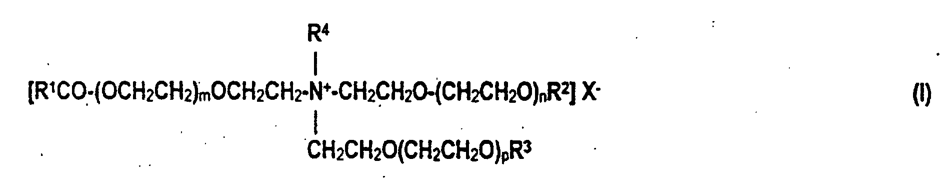 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Detergensgemische nach Anspruch 1, **dadurch gekennzeichnet, daß** sie Esterquats der Formel **(II)** enthalten, 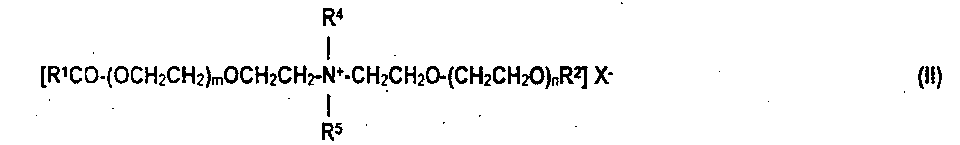 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Detergensgemische nach Anspruch 1, **dadurch gekennzeichnet, daß** sie Esterquats der Forme (III) enthalten, 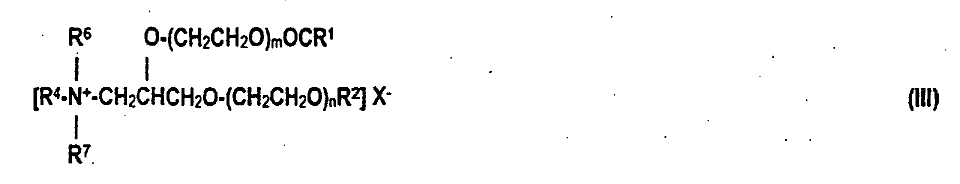 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Detergensgemische nach den Ansprüchen 1 bis 4, **dadurch gekennzeichnet, daß** sie Chitosan und/oder Chitosanderivate enthalten, die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

6. Detergensgemische nach den Ansprüchen 1 bis 5, **dadurch gekennzeichnet, daß** sie pflanzliche Proteinhydrolysate enthalten.

7. Detergensgemische nach den Ansprüchen 1 bis 6, **dadurch gekennzeichnet, daß** sie weiterhin Alkyl- und Alkenyloligoglykoside der Formel **(IV)** enthalten,
**R**^{**8**}**O-[G]**_{**p**} **(IV)**
in der R⁸ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

8. Detergensgemische nach den Ansprüchen 1 bis 7, **dadurch gekennzeichnet, daß** sie weiterhin Betaine der Formel **(V)** enthalten, 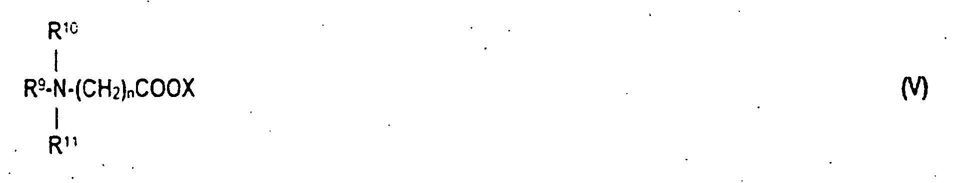 in der R⁹ für Alkyl- und/oder Alkenylreste mit 6 bis 22 Kohlenstoffatomen, R¹⁰ für **Wasserstoff oder** Alkylreste mit 1 bis 4 Kohlenstoffatomen, R¹¹ für Alkylreste mit 1 bis 4 Kohlenstoffatomen, n für Zahlen von 1 bis 6 und X für ein Alkali- und/oder Erdalkalimetall oder Ammonium steht.

9. Detergensgemische nach den Ansprüchen 1 bis 8, **dadurch gekennzeichnet, daß** sie weiterhin Betaine der Formel **(VI)** enthalten, 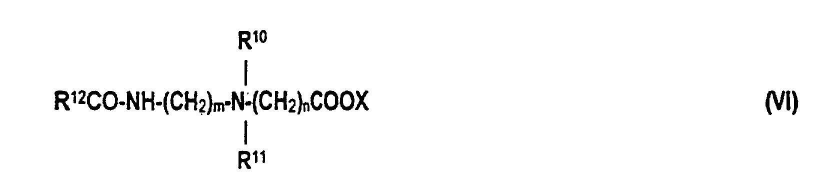 in der R¹²CO für einen aliphatischen Acylrest mit 6 bis 22 Kohlenstoffatomen und 0 oder 1 bis 3 Doppelbindungen, m für Zahlen von 1 bis 3 steht und R¹⁰, R¹¹, n und X die oben angegebenen Bedeutungen haben.

10. Verwendung von Detergensgemischen nach den Ansprüchen 1 bis 9 zur Herstellung oberflächenaktiver Mittel.

## Claims

1. Detergent mixtures containing
(a) esterquats,
(b) chitosan and/or chitosan derivatives and
(c) protein hydrolyzates.

2. Detergent mixtures as claimed in claim 1, **characterized in that** they contain esterquats corresponding to formula **(I):** 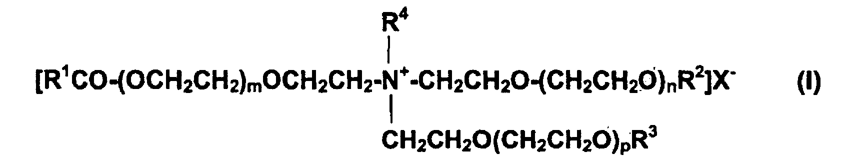 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate.

3. Detergent mixtures as claimed in claim 1, **characterized in that** they contain esterquats corresponding to formula **(II):** 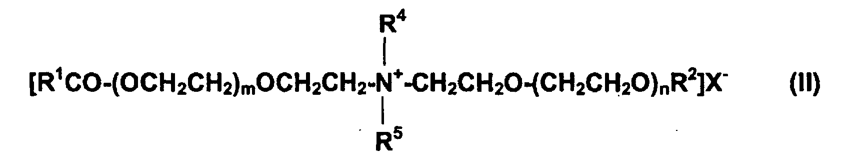 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

4. Detergent mixtures as claimed in claim 1, **characterized in that** they contain esterquats corresponding to formula **(III):** 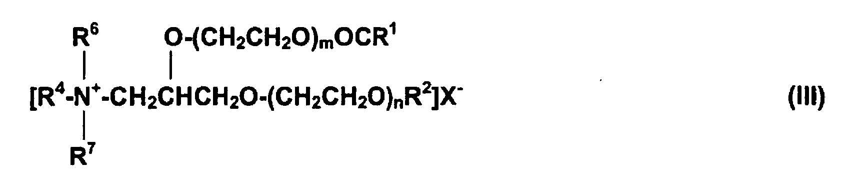 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

5. Detergent mixtures as claimed in claims 1 to 4, **characterized in that** they contain chitosan and/or chitosan derivatives which have an average molecular weight of 800,000 to 1,200,000 dalton, a Brookfield viscosity (1% by weight in glycolic acid) below 5000 mPas, a degree of deacetylation of 80 to 88% and an ash content of less than 0.3% by weight.

6. Detergent mixtures as claimed in claims 1 to 5, **characterized in that** they contain vegetable protein hydrolyzates.

7. Detergent mixtures as claimed in claims 1 to 6, **characterized in that** they additionally contain alkyl and alkenyloligoglycosides corresponding to formula **(IV):**
**R**^{**8**}**O-[G]**_{**p**} **(IV)**
in which R⁸ is an alkyl and/or alkenyl radical containing 4 to 22 carbon atoms, G is a sugar unit containing 5 or 6 carbon atoms and p is a number of 1 to 10.

8. Detergent mixtures as claimed in claims 1 to 7, **characterized in that** they additionally contain betaines corresponding to formula **(V)**: 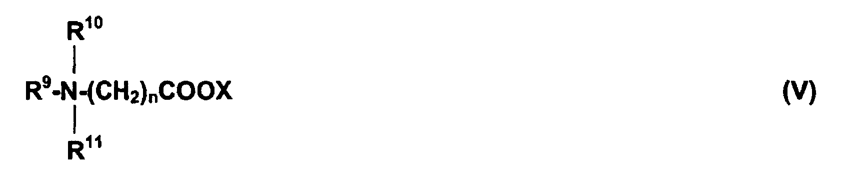 in which R⁹ represents alkyl and/or alkenyl groups containing 6 to 22 carbon atoms, R¹⁰ represents hydrogen or alkyl groups containing 1 to 4 carbon atoms, R¹¹ represents alkyl groups containing 1 to 4 carbon atoms, n is a number of 1 to 6 and X is an alkali metal and/or alkaline earth metal or ammonium.

9. Detergent mixtures as claimed in claims 1 to 8, **characterized in that** they additionally contain betaines corresponding to formula (VI): 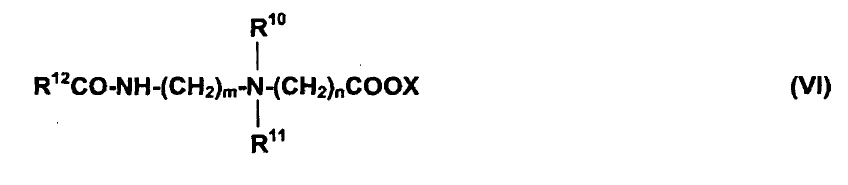 where R¹²CO is an aliphatic acyl group containing 6 to 22 carbon atoms and 0 or 1 to 3 double bonds, m is a number of 1 to 3 and R¹⁰, R¹¹, n and X are as defined above.

10. The use of the detergent mixtures claimed in claims 1 to 9 for the production of surface-active compositions.

## Revendications

1. Mélanges de détergents contenant :
a) des esterquats,
b) du chitosan et/ou des dérivés du chitosan et
c) des hydrolysats de protéine.

2. Mélanges de détergents selon la revendication 1,
**caractérisés en ce qu'**
ils renferment des esterquats de formule (I) 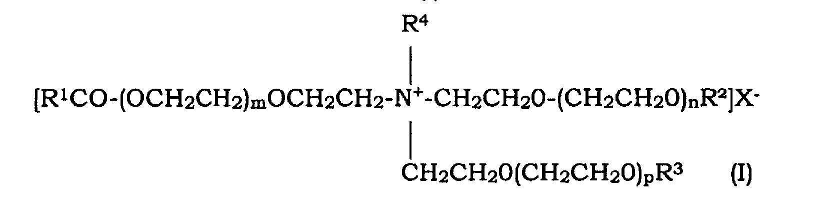 dans laquelle R¹CO représente un reste acyle ayant de 6 à 22 atomes de carbone, R² et R³ indépendamment l'un de l'autre représentent de l'hydrogène ou R¹CO, R⁴ représente un reste alkyle ayant de 1 à 4 atomes de carbone ou un groupe (CH₂ CH₂O)_{q}H, m, n et p globalement représentent O ou des nombres allant de 1 à 12, q représente des nombres allant de 1 à 12 et X représente un halogénure, un alkylsulfate ou un alkylphosphate.

3. Mélanges de détergents selon la revendication 1,
**caractérisés en ce qu'**
ils renferment des esterquats de formule (II) 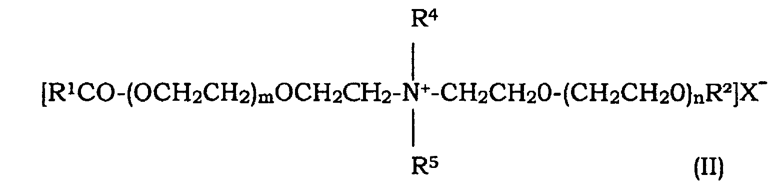 dans laquelle R¹CO représente un reste acyle ayant de 6 à 22 atomes de carbone, R² représente de l'hydrogène ou R¹CO, R⁴ et R⁵ indépendamment l'un de l'autre représentent des restes alkyle ayant de 1 à 4 atomes de carbone, m et n globalement représentent 0 ou des nombres allant de 1 à 12 et X représente un halogénure, un alkylsulfate ou un alkylphosphate.

4. Mélanges de détergents selon la revendication 1,
**caractérisés en ce qu'**
ils renferment des esterquats de formule (III) 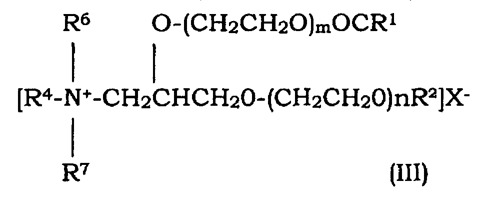 dans laquelle R¹CO représente un reste acyle ayant de 6 à 22 atomes de carbone, R² représente de l'hydrogène ou R¹CO, R⁴, R⁶ et R⁷ indépendamment les uns des autres, représentent des restes alkyle ayant de 1 à 4 atomes de carbone, m et n globalement représentent O ou des nombres allant de 1 à 12, et X représente un halogénure, un alkylsulfate ou un alkyl phosphate.

5. Mélanges de détergents selon les revendications 1 à 4,
**caractérisés en ce qu'**
ils renferment du chitosan et/ou des dérivés du chitosan qui possèdent
- un poids moléculaire moyen de 800.000 à 1 200.000 daltons.
- une viscosité selon Brookfield (à 1% en poids dans l'acide glycolique) en dessous de 5000 mPas,
- un degré de désacétylation dans la zone de 80 à 88 % et
- une teneur en cendres de moins de 0,3 % en poids.

6. Mélanges de détergents selon les revendications 1 à 5,
**caractérisés en ce qu'**
ils renferment des hydrolysats de protéines végétaux.

7. Mélanges de détergents selon les revendications 1 à 6,
**caractérisés en ce qu'**
ils contiennent en outre des alkyl - et des aikényi oligoglycosides de formule IV
R⁸O-[G]ₚ IV
dans laquelle R⁸ représente un reste alkyle et/ou aikényle ayant de 4 à 22 atomes de carbone, G représente un reste de sucre ayant 5 ou 6 atomes de carbone et p représente des nombres allant de 1 à 10.

8. Mélanges de détergents selon les revendications 1 à 7,
**caractérisés en ce qu'**
ils renferment en outre des bétaïnes de formule (V) 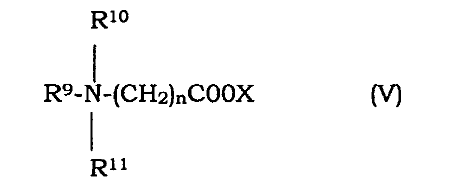 dans laquelle R⁹ représente des restes alkyle et/ou alkényle ayant de 6 à 22 atomes de carbone, R¹⁰ représente de l'hydrogène ou des restes alkyle ayant de 1 à 4 atomes de carbone, R¹¹ représente des restes alkyle ayant de 1 à 4 atomes de carbone, n représente des nombres allant de 1 à 6, et X représente un métal alcalin et/ou alcalinoferreux ou de l'ammonium.

9. Mélanges de détergents selon les revendications 1 à 8,
**caractérisés en ce qu'**
ils renferment en outre des bétaïnes de formule VI 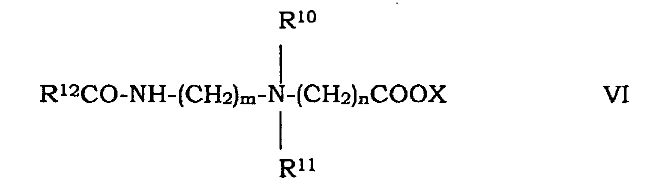 dans laquelle R¹²CO représente un reste acyle aliphatique ayant de 6 à 22 atomes de carbone, et 0 ou 1 à 3 double liaisons, m représente des nombres allant de 1 à 3 et R¹⁰, R¹¹, n et X ont les significations fournies ci-dessus.

10. Utilisation de mélanges de détergents selon les revendications 1 à 9, pour la production d'agents actifs sur la tension superficielle.
